# EUROPEAN PATENT SPECIFICATION

(11) **EP 3 077 058 B1**
(45) Date of publication and mention of the grant of the patent: **05.02.2020**
(21) Application number: 14805919.9
(22) Date of filing: 02.12.2014
(51) Int. Cl.: A61K 8/86, A61K 8/81, A61Q 5/06

(54) **COMPOSITION COMPRISING AT LEAST ONE ANIONIC ASSOCIATIVE POLYMER, AT LEAST ONE ANIONIC FIXING POLYMER AND AT LEAST ONE CATIONIC FIXING POLYMER**
KOSMETIKZUSAMMENSETZUNG MIT EINEM ANIONISCHEN ASSOZIATIVEN POLYMER, EINEM ANIONISCHEN FIXIERENDEN POLYMER UND EINEM KATIONISCHEN FIXIERENDEN POLYMER
COMPOSITION COMPRENANT AU MOINS UN POLYMERE ASSOCIATIF ANIONIQUE, AU MOINS UN POLYMERE FIXANT ANIONIQUE ET AU MOINS UN POLYMERE CATIONIQUE FIXANT.

(30) Priority: 02.12.2013 FR 1361948
(43) Date of publication of application: 12.10.2016
(73) Proprietor: L'Oréal, 75008 Paris (FR)
(72) Inventor: GILLES, Audrey, F-92110 Clichy (FR); BRAC DE LA PERRIERE, Anne-Sophie, 92600 Asnieres-Sur-Seine (FR)
(74) Representative: Casalonga
(86) International application number: PCT/EP2014/076258
(87) International publication number: WO 2015/082471

(56) References cited:
- DE-A1-102009 001 978
- FR-A1- 2 984 135

## Description

The present invention relates to a composition comprising one or more particular anionic associative polymers, one or more particular anionic fixing polymers and one or more cationic fixing polymers.

The present invention also relates to the use of the abovementioned composition for styling keratin fibres, preferably the hair.

Finally, the invention relates to a process for treating keratin fibres, preferably the hair, using the abovementioned composition.

Styling products are usually used to construct and structure the hairstyle and to give it long-lasting hold. These compositions generally comprise one or more fixing film-forming polymers, in a cosmetically acceptable medium. These polymers allow the formation of a coating film on the hair, thus providing form retention of the hairstyle.

Styling products are generally in the form of lacquers, foams or gels.

In particular, styling gels are often used in order to obtain strong fixing of the hairstyle.

Styling gels are solutions of one or more fixing film-forming polymers, thickened or gelled with one or more thickening polymers.

These thickening polymers are introduced in an attempt to give the composition good working qualities such as easy uptake in the hands, better distribution and good spreading on the hair.

However, the strong-fixing styling gels of the prior art generally have several drawbacks, namely having an uptake in the hands that is not optimal and giving insufficient fixing and also insufficient hold of the hairstyle over time.

In particular, the fixing capacities of these gels tend to reach an upper limit, which prohibits styling gels with very strong fixing.

Finally, residue that is visible on the user's hair, scalp and/or clothes is often observed, after removal of the styling gel.

There is thus a need to formulate a composition, especially a strong-fixing styling composition, which ensures improved working qualities and also durability of the shaping and which leaves little or no residue visible on the user's hair, scalp and/or clothes, after its removal.

It has now been discovered that the combination of one or more particular anionic associative polymers, one or more particular anionic fixing polymers and one or more cationic fixing polymers makes it possible to obtain a hair composition that has improved working qualities, substantial fixing qualities and high styling performance over time.

One subject of the present invention is thus especially a composition comprising:
i) one or more anionic associative polymers,
ii) one or more anionic fixing polymers,
iii) one or more cationic fixing polymers chosen from copolymers derived from acrylic or methacrylic esters comprising at least one unit of formulae (VI) and (VII) as defined hereafter and at least one unit derived from one or more comonomers chosen from vinyllactams,
the said anionic associative polymer(s) comprising at least one unit of formula (I) and at least one unit of formula (II) below: 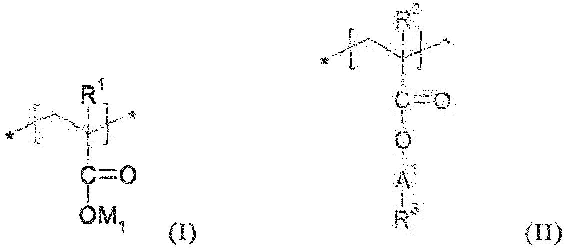
with R¹ and R², independently of each other, representing a hydrogen atom or a methyl group,
R³ representing a C₈-C₃₀ alkyl radical,
M₁ representing a hydrogen atom or a physiologically acceptable cation,
A¹ representing a group -(CH₂CH₂O)ₓ-, x being an integer ranging from 5 to 35, a group -(CH₂CHMeO)_{y}-, y being an integer ranging from 5 to 35, or a group -(CH₂CH₂O)ₓ-(CH₂CHMeO)_{y}-, the sum x + y being an integer ranging from 5 to 35 with x and y being greater than 0, and
the said anionic fixing polymer(s) comprising at least one unit of formula (III) and at least one unit of formula (IV) below: 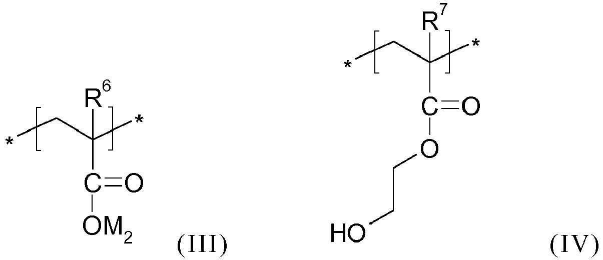
   with R⁶ and R⁷, independently of each other, representing a hydrogen atom or a methyl group, and
   M₂ representing a hydrogen atom or a physiologically acceptable cation.

The composition according to the invention has improved working qualities, and especially easy uptake in the hands and better distribution and good spreading of the composition on the hair.

Moreover, the composition according to the invention also has high styling performance, and in particular great fixing power and long-lasting hold of the hairstyle treated with this composition.

Finally, the composition according to the invention leaves little or no residue visible on the user's hair, scalp and/or clothes, once it has been removed.

A subject of the present invention is also the use of the abovementioned composition for styling keratin fibres, preferably the hair.

Finally, a subject of the invention is a process for treating keratin fibres, preferably the hair, comprising the application of the abovementioned composition to the keratin fibres.

Other subjects, characteristics, aspects and advantages of the invention will emerge even more clearly on reading the description and the examples that follow.

In that which follows, and unless otherwise indicated, the limits of a range of values are included within this range, in particular in the expressions "of between" and "ranging from ... to ...".

In addition, the expression "at least one" is equivalent to the expression "one or more".

For the purposes of the present invention, the term "associative polymer" means an amphiphilic polymer that is capable, in an aqueous medium, of reversibly combining with itself or with other molecules. It generally comprises, in its chemical structure, at least one hydrophilic region or group and at least one hydrophobic region or group.

For the purposes of the present invention, the term "hydrophobic group" means a group or a polymer bearing a saturated or unsaturated and linear or branched hydrocarbon-based chain.

When it denotes a hydrocarbon-based group, the hydrophobic group comprises at least 8 carbon atoms, preferably from 10 to 30 carbon atoms, in particular from 12 to 30 carbon atoms and preferentially from 18 to 30 carbon atoms. Preferentially, the hydrocarbon-based hydrophobic group originates from a monofunctional compound. By way of example, the hydrophobic group may be derived from a fatty alcohol, such as stearyl alcohol, dodecyl alcohol or decyl alcohol, or else from a polyalkylenated fatty alcohol, such as Steareth-100.

It may also denote a hydrocarbon-based polymer, for instance polybutadiene.

For the purposes of the present invention, the term "fixing polymer" means a polymer that is capable of giving a head of hair a shape and/or of holding the head of hair in a given shape.

As explained previously, the composition according to the invention comprises one or more particular anionic associative polymers (i) comprising at least one unit of formula (I) and at least one unit of formula (II) below: 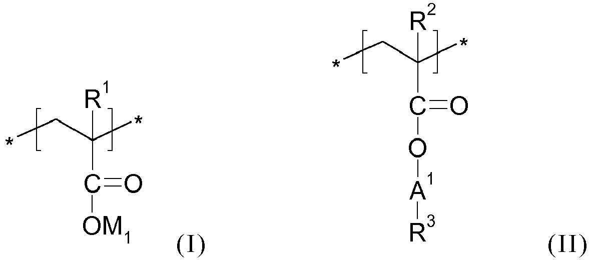
with R¹ and R², independently of each other, representing a hydrogen atom or a methyl group,
R³ representing a C₈-C₃₀ alkyl radical,
M₁ representing a hydrogen atom or a physiologically acceptable cation, and
A¹ representing a group -(CH₂CH₂O)ₓ-, x being an integer ranging from 5 to 35, a group -(CH₂CHMeO)_{y}-, y being an integer ranging from 5 to 35, or a group -(CH₂CH₂O)ₓ-(CH₂CHMeO)_{y}-, the sum x + y being an integer ranging from 5 to 35 with x and y being greater than 0.
As C₈ to C₃₀ alkyl radical according to the invention, mention may be made especially of the following radicals: octyl (capryl), decyl (caprinyl), dodecyl (lauryl), tetradecyl (myristyl), hexadecyl (cetyl), octadecyl (stearyl), eicosyl (arachidyl) and docosyl (behenyl).

In the above formulae and in all the following formulae, a chemical bond indicated by the symbol * corresponds to a free valency of the structural fragment in question.

As physiologically acceptable cation in formula (I) above and also in the other formulae of the description, mention may be made especially of cations of the physiologically acceptable metals of groups la, Ib, IIa, IIb, IIIb, VIa and VIII of the Periodic Table of the Elements, ions of ammonium type and cationic organic compounds comprising a quaternized nitrogen atom. The latter are formed, for example, by protonation, using an acid, of a primary, secondary or tertiary organic amine, or by permanent quaternization of such organic amines. Examples of these organic cationic ammonium compounds are 2-ammonioethanol and 2-trimethylammonioethanol.

Preferably, the physiologically acceptable cation in formula (I) above and in the other formulae of the description is chosen from a sodium ion and an ion derived from an alkanolamine.

Preferably, M₁ represents a hydrogen atom, a sodium ion or an ion derived from an alkanolamine.

In a first embodiment of the invention, the anionic associative polymer(s) that may be used in the composition according to the invention are chosen from those in which, in formula (II), A¹ represents a group of formula -(CH₂CH₂O)ₓ-, x being an integer ranging from 5 to 35 and in particular ranging from 10 to 24.

In a second embodiment of the invention, the anionic associative polymer(s) comprise a unit of formula (II) above in which R³ represents a C₁₂-C₂₀ alkyl radical.

In a third embodiment of the invention, the anionic associative polymer(s) that may be used in the composition according to the invention are chosen from those in which, in formula (II), R² represents a methyl group.

In a fourth embodiment of the invention, the anionic associative polymer(s) comprise at least one unit of formula (I) as defined above and at least one unit of formula (II') below: 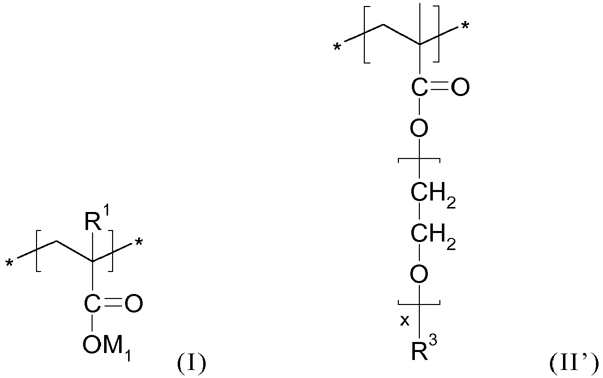 with R¹ representing a hydrogen atom or a methyl group, M₁ representing a hydrogen atom or a physiologically acceptable cation, R³ representing a C₈ to C₃₀ alkyl radical and x being an integer ranging from 5 to 35 and preferably ranging from 10 to 24

Preferably, in the composition according to the invention, the anionic associative polymer(s) are crosslinked.

For the purposes of the invention, the terms "crosslinked" and "crosslinking" should be understood as meaning the interconnection of the polymer chains via covalent chemical bonds, with formation of a network. This covalent connection of the polymer chains may be performed via direct covalent bonds or by means of molecular fragments forming bridges between the polymer chains. Such a molecular fragment is linked via a covalent chemical bond to each of the polymer chains between which it forms a bridge. For the purposes of the invention, the term "non-crosslinked" should be understood as meaning the absence of any "crosslinking" as defined above.

Crosslinking of the anionic associative polymer(s) is generally performed using at least one crosslinking agent.

The crosslinking agent(s) may be chosen especially from polyunsaturated aromatic compounds such as divinylbenzenes, divinylnaphthalenes and trivinylbenzenes, polyunsaturated alicyclic compounds such as 1,2,4-trivinylcyclohexane, phthalic acid diesters such as diallyl phthalate, polyunsaturated aliphatic compounds such as dienes, trienes and tetraenes, for instance isoprene, 1,3-butadiene, 1,5-hexadiene, 1,5,9-decatriene, 1,9-decadiene and 1,5-heptadiene, polyalkenyl ethers such as triallylpentaerythritol, diallylpentaerythritol, diallylsaccharose, octaallylsaccharose and trimethylolpropane diallyl ether, polyunsaturated esters of polyols or of polyacids such as 1,6-hexanediol di(meth)acrylate, tetramethylolmethane tri(meth)acrylate, allyl acrylate, diallyl itaconate, diallyl fumarate, diallyl maleate, trimethylolpropane tri(meth)acrylate, trimethylolpropane di(meth)acrylate and polyethylene glycol di(meth)acrylate such as ethylene glycol di(meth)acrylate, diethylene glycol di(meth)acrylate and triethylene glycol di(meth)acrylate, alkylenebisacrylamides such as methylenebisacrylamide and propylenebisacrylamide, hydroxyl or carboxyl derivatives of methylenebisacrylamide such as N,N'-bis(methylol)methylenebisacrylamide, polyunsaturated silanes such as dimethyldivinylsilane, methyltrivinylsilane, allyldimethylvinylsilane, diallyldimethylsilane and tetravinylsilane, N-methylolacrylamide, N-alkoxy(meth)acrylamides in which the alkoxy group is a C₁-C₁₈ alkoxy group, hydrolysable unsaturated silanes such as triethoxyvinylsilane, triisopropoxyvinylsilane and 3-(triethoxysilyl)propyl methacrylate, hydrolysable silanes such as ethyltriethoxysilane and ethyltrimethoxysilane, hydrolysable silanes bearing an epoxy substituent such as [2-(3,4-epoxycyclohexyl)ethyl]triethoxysilane and (3-glycidyloxypropyl)trimethoxysilane, polyisocyanates such as butane 1,4-diisocyanate, hexane 1,6-diisocyanate, phenylene 1,4-diisocyanate and 4,4'-oxybis(phenyl isocyanate), unsaturated epoxides such as glycidyl methacrylate and allyl glycidyl ether, polyepoxides such as 1,2,5,6-diepoxyhexane, diglycidyl ether and ethylene glycol diglycidyl ether, ethoxylated polyols such as diols, triols or diphenols, in each case ethoxylated with 2 to 100 mol of ethylene oxide per mole of hydroxyl groups and bearing, as end group, a polymerizable unsaturated group, for instance a group of vinyl ether, allyl ether, acrylate ester or methacrylate ester type, and acrylate and methacrylate esters of polyols comprising at least two functional groups of acrylate or methacrylate ester type such as trimethylolpropane triacrylate (TMPTA), 15-ethoxylated trimethylolpropane triacrylate (TMPEO15TA), trimethylolpropane dimethacrylate, triethylene glycol dimethacrylate (TEGDMA) and bisphenol A dimethacrylate ethoxylated with 30 mol of ethylene oxide (EOBDMA). Ethoxylated polyols that may especially be mentioned include ethoxylated bisphenol A di(meth)acrylate, ethoxylated bisphenol F di(meth)acrylate and ethoxylated trimethylolpropane tri(meth)acrylate.

In a preferred variant of the invention, the anionic associative polymer(s) comprise, in addition to at least one unit of formula (I) and at least one unit of formula (II), at least one unit of formula (V) below: 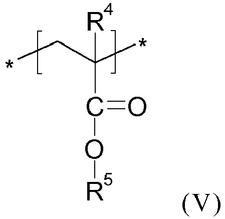
with R⁴ representing a hydrogen atom or a methyl group, and
R⁵ representing a C₁ to C₄ alkyl group, and in particular a methyl or ethyl group. Preferably, R⁵ represents an ethyl group.

In a particularly preferred embodiment of this variant, the anionic associative polymer(s) preferably comprise at least one unit of formula (I) as defined above, at least one unit of formula (II') as defined above and at least one unit of formula (V) as defined above, with R¹ and R⁴, independently of each other, representing a hydrogen atom or a methyl group, R³ representing a C₈ to C₃₀ alkyl group, R⁵ representing a C₁ to C₄ alkyl group, preferably an ethyl or methyl group, M₁ representing a hydrogen atom or a physiologically acceptable cation, and x being an integer ranging from 5 to 35 and preferably ranging from 10 to 24.

Preferably, M₁ represents a hydrogen atom, a sodium ion or an ion derived from an alkanolamine.

Preferably, an anionic associative polymer that is most particularly suitable for use in the composition according to the invention is the acrylate/steareth-20 methacrylate crosslinked copolymer (INCI name: acrylate/steareth-20 methacrylate crosspolymer) which comprises 20 ethylene oxide units. Such a polymer is sold, for example, by the company Röhm & Haas, in the form of a dispersion at 28-30% by weight in water, under the brand name Aculyn® 88.

The particular anionic associative polymer(s) (i) may represent from 0.1% to 20% by weight, preferably from 0.5% to 15% by weight, more preferentially from 1% to 10% by weight and in particular from 1% to 5% by weight, relative to the total weight of the composition.

As explained previously, the composition according to the invention also comprises one or more anionic fixing polymers (ii) comprising at least one unit of formula (III) and at least one unit of formula (IV) below: 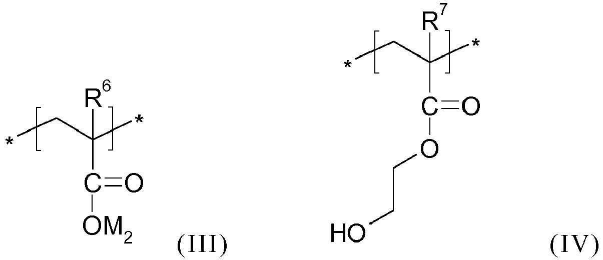
with R⁶ and R⁷, independently of each other, representing a hydrogen atom or a methyl group, and
M₂ representing a hydrogen atom or a physiologically acceptable cation.

Preferably, M₂ represents a hydrogen atom, a sodium ion or an ion derived from an alkanolamine.

In a preferred variant of the invention, the anionic fixing polymer(s) also comprise at least one unit of formula (III) and at least one unit of formula (IV), at least one unit of formula (V) as defined above, with R⁴ representing a hydrogen atom or a methyl group, and R⁵ representing a C₁ to C₄ alkyl group, in particular a methyl, ethyl or n-butyl group.

These anionic fixing polymers (ii) are different from the anionic associative polymers (i).

Preferably, in the composition according to the invention, the anionic fixing polymer(s) are non-crosslinked.

Preferably, the anionic fixing polymer that may be used in the composition according to the invention is a copolymer of methacrylic acid/hydroxyethyl methacrylate and of at least one C₁ to C₄ (meth)acrylic acid ester.

In a most particularly preferred manner, the anionic fixing polymer that may be used in the composition according to the invention is a methacrylic acid/hydroxyethyl methacrylate/methyl methacrylate/butyl acrylate linear tetrapolymer.

Such a polymer is known especially under the trade name Acudyne® 180 and is sold by the company Röhm & Haas.

The anionic fixing polymer(s) (ii) may represent from 0.1% to 20% by weight, preferably from 0.2% to 15% by weight, more preferentially from 0.5% to 10% by weight and in particular from 1% to 8% by weight, relative to the total weight of the composition.

Preferably, in the composition according to the invention, the weight ratio of the anionic associative polymer(s) to the anionic fixing polymer(s) ranges from 0.2 to 5, preferably from 0.5 to 5 and in particular from 0.6 to 2.

As explained previously, the composition according to the invention also comprises one or more cationic fixing polymers.

For the purposes of the present invention, the term "cationic polymer" means any polymer containing cationic groups and/or groups that may be ionized into cationic groups, which are preferably not siliceous.

The cationic fixing polymer(s) that may be used in the composition according to the present invention are preferably chosen from polymers comprising primary, secondary, tertiary and/or quaternary amine groups forming part of the polymer chain or directly connected thereto, and having a molecular weight of between 500 and about 5 000 000 and preferably between 1000 and 3 000 000.

The cationic fixing polymers are chosen from copolymers derived from acrylic or methacrylic esters, and comprising: at least one of the units of formulae (VI)) and (VII) below: 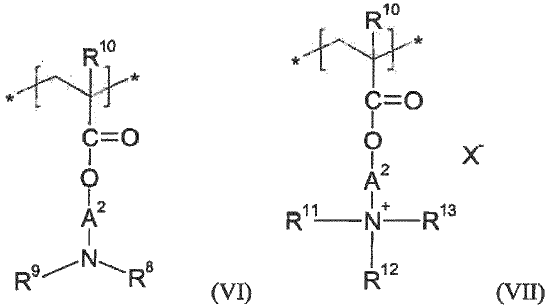 in which:
R⁸ and R⁹, which may be identical or different, represent a hydrogen atom or an alkyl group containing from 1 to 6 carbon atoms;
R¹⁰, which may be identical or different in each formula, denotes a hydrogen atom or a CH₃ radical;
A², which may be identical or different in each formula, denotes a linear or branched alkyl group comprising 1 to 6 carbon atoms or a hydroxyalkyl group comprising 1 to 4 carbon atoms;
R¹¹, R¹² and R¹³, which may be identical or different in each formula, represent an alkyl group containing from 1 to 18 carbon atoms or a benzyl radical;
X⁻ denotes a methosulfate anion or a halide such as chloride or bromide.

The copolymers of family (1) also contain one or more comonomer units that are chosen from vinyllactams such as vinylpyrrolidone or vinylcaprolactam.

Thus, among these copolymers of family (1), mention may be made of:
- quaternized or non-quaternized vinylpyrrolidone/dialkylaminoalkyl acrylate or methacrylate copolymers, such as the products sold under the name Gafquat® by the company ISP, such as, for example, Gafquat® 734 or Gafquat® 755 or alternatively the products known as Copolymer® 845, 958 and 937. These polymers are described in detail in French patents 2 077 143 and 2 393 573,
- fatty-chain polymers containing a vinylpyrrolidone unit, such as the products sold under the name Styleze W20 and Styleze W10 by the company ISP,
- dimethylaminoethyl methacrylate/vinylcaprolactam/vinylpyrrolidone terpolymers, such as the product sold under the name Gaffix VC 713 by the company ISP, and
- quaternized vinylpyrrolidone/dimethylaminopropylmethacrylamide copolymers, such as the product sold under the name Gafquat® HS 100 by the company ISP;

The cationic fixing polymers are copolymers derived from acrylic or methacrylic esters comprising at least one unit of formulae (VI) and (VII) as defined previously and at least one unit derived from one or more comonomers chosen from vinyllactams.

More particularly, the cationic fixing polymers are chosen from quaternized or non-quaternized, preferably non-quaternized, vinylpyrrolidone/dialkylaminoalkyl acrylate or methacrylate copolymers, especially those sold under the trade name Copolymer® 845 by ISP.

The cationic fixing polymer(s) may represent from 0.1% to 40% by weight, preferably from 1% to 30% by weight and in particular from 2% to 20% by weight, relative to the total weight of the composition.

According to one embodiment, the composition according to the present invention comprises:
i) one or more anionic associative polymers containing at least one unit of formula (I) as defined above, at least one unit of formula (II') as defined above and at least one unit of formula (V) as defined above,
ii) one or more anionic fixing polymers containing at least one unit of formula (III) as defined above, at least one unit of formula (IV) as defined above and at least one unit of formula (V) as defined above,
iii) one or more cationic fixing polymers chosen from quaternized or non-quaternized, preferably non-quaternized, vinylpyrrolidone/dialkylaminoalkyl acrylate or methacrylate copolymers.

According to a preferred embodiment, the composition according to the present invention may also comprise one or more surfactants chosen from anionic surfactants, amphoteric or zwitterionic surfactants, nonionic surfactants and cationic surfactants.

The term "anionic surfactant" means a surfactant comprising, as ionic or ionizable groups, only anionic groups. These anionic groups are preferably chosen from the groups CO₂H, CO₂⁻, SO₃H, SO₃⁻, OSO₃H, OSO₃⁻, H₂PO₃, HPO₃⁻, PO₃²⁻, H₂PO₂, HPO₂⁻, PO₂²⁻, POH and PO⁻.

As examples of anionic surfactants that may be used in the composition according to the invention, mention may be made of alkyl sulfates, alkyl ether sulfates, alkylamido ether sulfates, alkylarylpolyether sulfates, monoglyceride sulfates, alkylsulfonates, alkylamidesulfonates, alkylarylsulfonates, α-olefin sulfonates, paraffin sulfonates, alkylsulfosuccinates, alkylether sulfosuccinates, alkylamide sulfosuccinates, alkylsulfoacetates, acylsarcosinates, acylglutamates, alkylsulfosuccinamates, acylisethionates and N-(C₁-C₄)alkyl N-acyltaurates, salts of alkyl monoesters and of polyglycoside-polycarboxylic acids, acyllactylates, D-galactoside uronic acid salts, alkyl ether carboxylic acid salts, alkylaryl ether carboxylic acid salts, alkylamido ether carboxylic acid salts; and the corresponding non-salified forms of all these compounds; the alkyl and acyl groups of all these compounds (unless otherwise mentioned) generally comprising from 6 to 24 carbon atoms and the aryl group generally denoting a phenyl group.

These compounds can be oxyethylenated and then preferably comprise from 1 to 50 ethylene oxide units.

The salts of C₆-C₂₄ alkyl monoesters of polyglycoside-polycarboxylic acids can be chosen from C₆-C₂₄ alkyl polyglycoside-citrates, C₆-C₂₄ alkyl polyglycoside-tartrates and C₆-C₂₄ alkyl polyglycoside-sulfo succinates.

When the anionic surfactant(s) are in salt form, they may be chosen from alkali metal salts such as the sodium or potassium salt and preferably the sodium salt, ammonium salts, amine salts and in particular amino alcohol salts or alkaline-earth metal salts such as the magnesium salt.

Examples of amino alcohol salts that may especially be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts.

Use is preferably made of alkali metal or alkaline-earth metal salts, in particular the sodium or magnesium salts.

The anionic surfactants that may be present may be mild anionic surfactants, i.e. anionic surfactants without a sulfate function.

Mention may in particular be made, as regards the mild anionic surfactants, of the following compounds and salts thereof, and also mixtures thereof:
polyoxyalkylenated alkyl ether carboxylic acids;
polyoxyalkylenated alkylaryl ether carboxylic acids;
polyoxyalkylenated alkylamido ether carboxylic acids, in particular those comprising 2 to 50 ethylene oxide groups;
alkyl-D-galactoside uronic acids;
acylsarcosinates, acylglutamates; and
alkylpolyglycoside carboxylic esters.

Use may be made most particularly of polyoxyalkylenated alkyl ether carboxylic acids, for instance lauryl ether carboxylic acid (4.5 OE) sold, for example, under the name Akypo RLM 45 CA from Kao.

The amphoteric or zwitterionic surfactant(s) that may be used in the present invention may especially be secondary or tertiary aliphatic amine derivatives, optionally quaternized, in which the aliphatic group is a linear or branched chain containing from 8 to 22 carbon atoms, the said amine derivatives containing at least one anionic group, for instance a carboxylate, sulfonate, sulfate, phosphate or phosphonate group.

Mention may be made in particular of (C₈-C₂₀)alkylbetaines, sulfobetaines, (C₈-C₂₀)alkylamido(C₃-C₈)alkylbetaines or (C₈-C₂₀)alkylamido(C₆-C₈)alkylsulfobetaines.

Among the secondary or tertiary aliphatic amine derivatives, optionally quaternized, that may be used, as defined above, mention may also be made of the compounds of respective structures (A₁), (A₂) and (A₃) below:

Rₐ-CONHCH₂CH₂-N⁺(R_{b})(R_{c})(CH₂COO⁻) (A₁)

in which:
Rₐ represents a C₁₀-C₃₀ alkyl or alkenyl group derived from an acid Rₐ-COOH preferably present in hydrolysed coconut oil, or a heptyl, nonyl or undecyl group,
R_{b} represents a β-hydroxyethyl group, and
R_{c} represents a carboxymethyl group;
and

R'ₐ-CONHCH₂CH₂-N(B)(B') (A₂)

in which:
B represents -CH₂CH₂OX',
B' represents -(CH₂)_{z}-Y', with z = 1 or 2,
X' represents the -CH₂-COOH, CH₂-COOZ', -CH₂CH₂-COOH or -CH₂CH₂-COOZ' group, or a hydrogen atom,
Y' represents -COOH, -COOZ', or the group -CH₂-CHOH-SO₃H or CH₂-CHOH-SO₃Z',
Z' represents an ion resulting from an alkali metal or alkaline-earth metal, such as sodium, an ammonium ion or an ion resulting from an organic amine,
R'ₐ represents a C₁₀ to C₃₀ alkyl or alkenyl group of an acid R'ₐ-COOH which is preferably present in coconut oil or in hydrolysed linseed oil, or an alkyl group, especially a C₁₇ group, and its iso form, or an unsaturated C₁₇ group.

These compounds of formula (A₁) or (A₂) are classified in the CTFA dictionary, 5th edition, 1993, under the names disodium cocoamphodiacetate, disodium lauroamphodiacetate, disodium caprylamphodiacetate, disodium caprylamphodiacetate, disodium cocoamphodipropionate, disodium lauroamphodipropionate, disodium caprylamphodipropionate, disodium caprylamphodipropionate, lauroamphodipropionic acid, and cocoamphodipropionic acid.

By way of example, mention may be made of the cocoamphodiacetate sold by the company Rhodia under the trade name Miranol® C2M Concentrate.

Rₐ"-NHCH(Y")-(CH₂)ₙCONH(CH₂)_{n'}-N(R_{d})(Rₑ) (A₃)

in which formula:
Y" represents the group -COOH, -COOZ", -CH₂-CH(OH)SO₃H or the group -CH₂CH(OH)SO₃-Z";
R_{d} and Rₑ represent, independently of each other, a C₁-C₄ alkyl or hydroxyalkyl radical;
Z" represents a cationic counterion derived from an alkali metal or alkaline-earth metal, such as sodium, an ammonium ion or an ion derived from an organic amine;
Rₐ" represents a C₁₀-C₃₀ alkyl or alkenyl group of an acid Rₐ"-COOH which is preferably present in coconut oil or in hydrolysed linseed oil;
n and n' denote, independently of each other, an integer ranging from 1 to 3.

Among the compounds of formula (A₃), mention may be made of the compound classified in the CTFA dictionary under the name sodium diethylaminopropyl cocoaspartamide and sold by the company Chimex under the name Chimexane HB.

Among the abovementioned amphoteric or zwitterionic surfactants, it is preferred to use (C₈-C₂₀) alkylbetaines such as cocoylbetaine, (C₈-C₂₀) alkylamido(C₃-C₈) alkylbetaines such as cocoylamidopropylbetaine, and mixtures thereof. More preferentially, the amphoteric or zwitterionic surfactant(s) are chosen from cocoylamidopropylbetaine and cocoylbetaine.

The nonionic surfactant(s) in the compositions of the present invention are especially described, for example, in the Handbook of Surfactants by M.R. Porter, published by Blackie & Son (Glasgow and London), 1991, pp. 116-178. They are especially chosen from fatty alcohols, fatty α-diols, fatty (C₁-C₂₀)alkylphenols and fatty acids, these compounds being ethoxylated, propoxylated or glycerolated and containing at least one fatty chain comprising, for example, from 8 to 18 carbon atoms, the number of ethylene oxide or propylene oxide groups possibly ranging especially from 1 to 200, and the number of glycerol groups possibly ranging especially from 1 to 30.

Mention may also be made of condensates of ethylene oxide and of propylene oxide with fatty alcohols, ethoxylated fatty amides preferably having from 1 to 30 ethylene oxide units, polyglycerolated fatty amides comprising on average from 1 to 5 glycerol groups, and in particular from 1.5 to 4, ethoxylated fatty acid esters of sorbitan containing from 1 to 30 ethylene oxide units, fatty acid esters of sucrose, fatty acid esters of polyethylene glycol, (C₆-C₂₄)alkylpolyglycosides, oxyethylenated plant oils, N-(C₆-C₂₄)alkylglucamine derivatives, amine oxides such as (C₁₀-C₁₄)alkylamine oxides or N-(C₁₀-C₁₄)acylaminopropylmorpholine oxides.

The cationic surfactant(s) that may be used in the composition according to the invention are generally chosen from optionally polyoxyalkylenated primary, secondary or tertiary fatty amine salts, quaternary ammonium salts, and mixtures thereof.

Examples of quaternary ammonium salts that may especially be mentioned include:
- those corresponding to the general formula (IX) below: 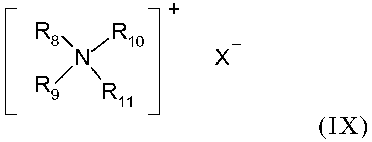 in which the groups R₈ to R₁₁, which can be identical or different, represent a linear or branched aliphatic group comprising from 1 to 30 carbon atoms or an aromatic group, such as aryl or alkylaryl, at least one of the groups R₈ to R₁₁ comprising from 8 to 30 carbon atoms and preferably from 12 to 24 carbon atoms. The aliphatic groups can comprise heteroatoms, such as, in particular, oxygen, nitrogen, sulfur and halogens.

The aliphatic groups are chosen, for example, from C₁-C₃₀ alkyl, C₁-C₃₀ alkoxy, polyoxy(C₂-C₆)alkylene, C₁-C₃₀ alkylamide, (C₁₂-C₂₂)alkylamido(C₂-C₆)alkyl, (C₁₂-C₂₂)alkyl acetate, and C₁-C₃₀ hydroxyalkyl groups, X⁻ is an anion chosen from the group of halides, phosphates, acetates, lactates, (C₁-C₄)alkyl sulfates, and (C₁-C₄)alkyl- or (C₁-C₄)alkylarylsulfonates.

Preference is given, among the quaternary ammonium salts of formula (IX), on the one hand, to tetraalkylammonium chlorides, such as, for example, dialkyldimethylammonium or alkyltrimethylammonium chlorides in which the alkyl group comprises approximately from 12 to 22 carbon atoms, in particular behenyltrimethylammonium chloride, distearyldimethylammonium chloride, cetyltrimethylammonium chloride or benzyldimethylstearylammonium chloride, or also, on the other hand, to distearoylethylhydroxyethylmethylammonium methosulfate, dipalmitoylethylhydroxyethylammonium methosulfate or distearoylethylhydroxyethylammonium methosulfate, or also, finally, to palmitylamidopropyltrimethylammonium chloride or stearamidopropyldimethyl(myristyl acetate)ammonium chloride, sold under the name Ceraphyl® 70 by the company Van Dyk.
- quaternary ammonium salts of imidazoline, for instance those of formula (X) below: 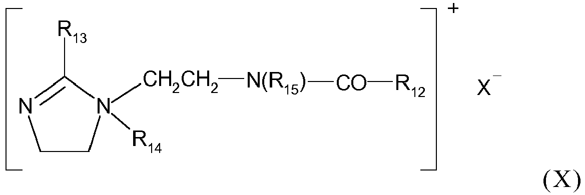 in which
   R₁₂ represents an alkenyl or alkyl group comprising from 8 to 30 carbon atoms, for example derived from tallow fatty acids,
   R₁₃ represents a hydrogen atom, a C₁-C₄ alkyl group or an alkenyl or alkyl group comprising from 8 to 30 carbon atoms,
   R₁₄ represents a C₁-C₄ alkyl group,
   R₁₅ represents a hydrogen atom, a C₁-C₄ alkyl group, X⁻ is an anion chosen from the group of halides, phosphates, acetates, lactates, (C₁-C₄)alkyl sulfates and (C₁-C₄)alkyl- or (C₁-C₄)alkylarylsulfonates.

Preferably, R₁₂ and R₁₃ denote a mixture of alkenyl or alkyl groups comprising from 12 to 21 carbon atoms, for example derived from tallow fatty acids, R₁₄ denotes a methyl group and R₁₅ denotes a hydrogen atom. Such a product is sold, for example, under the name Rewoquat® W 75 by the company Rewo.
- quaternary diammonium or triammonium salts, particularly of formula (XI) below: 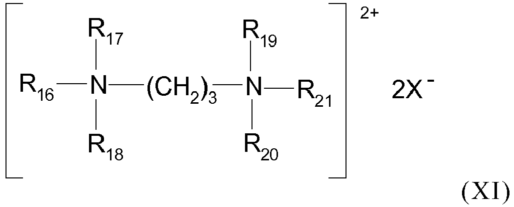
   in which R₁₆ denotes an alkyl group comprising approximately from 16 to 30 carbon atoms, which is optionally hydroxylated and/or interrupted with one or more oxygen atoms,
   R₁₇ is chosen from hydrogen, an alkyl group comprising from 1 to 4 carbon atoms or a group -(CH₂)₃-N⁺(R₁₆ₐ)(R₁₇ₐ)(R₁₈ₐ),
   R₁₆ₐ, R₁₇ₐ, R₁₈ₐ, R₁₈, R₁₉, R₂₀ and R₂₁, which may be identical or different, are chosen from hydrogen and an alkyl group comprising from 1 to 4 carbon atoms, and
   X⁻ is an anion chosen from the group of halides, acetates, phosphates, nitrates, (C₁-C₄)alkyl sulfates, (C₁-C₄)alkyl- or (C₁-C₄)alkylarylsulfonates, in particular methyl sulfate and ethyl sulfate.

Such compounds are, for example, Finquat CT-P, sold by the company Finetex (Quaternium 89), and Finquat CT, sold by the company Finetex (Quaternium 75);
- quaternary ammonium salts comprising one or more ester functions, such as those of formula (XII) below: 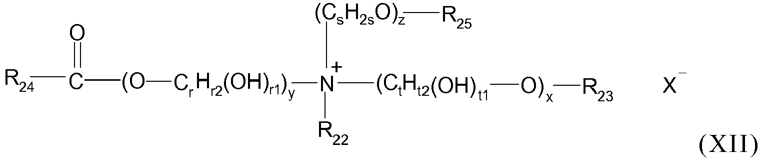 in which:
   R₂₂ is chosen from C₁-C₆ alkyl groups and C₁-C₆ hydroxyalkyl or dihydroxyalkyl groups,
   R₂₃ is chosen from:
      - the group 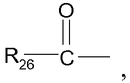
      - saturated or unsaturated and linear or branched C₁-C₂₂ hydrocarbon-based groups R₂₇,
      - a hydrogen atom,
   R₂₅ is chosen from:
      - the group 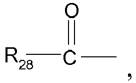
      - saturated or unsaturated and linear or branched C₁-C₆ hydrocarbon-based groups R₂₉,
      - a hydrogen atom,
   R₂₄, R₂₆ and R₂₈, which may be identical or different, are chosen from linear or branched, saturated or unsaturated C₇-C₂₁ hydrocarbon groups,
   r, s and t, which may be identical or different, are integers ranging from 2 to 6,
   r1 and t1, which are identical or different, have the values 0 or 1,
   r2 + r1 = 2 r and t1 + t2 = 2 t,
   y is an integer ranging from 1 to 10,
   x and z, which may be identical or different, are integers ranging from 0 to 10,
   X⁻ is a simple or complex, organic or mineral anion,
   with the proviso that the sum x + y + z is from 1 to 15, that when x is 0 then R₂₃ denotes R₂₇, and that when z is 0 then R₂₅ denotes R₂₉.

The alkyl groups R₂₂ may be linear or branched, and more particularly linear.

Preferably, R₂₂ denotes a methyl, ethyl, hydroxyethyl or dihydroxypropyl group and more particularly a methyl or ethyl group.

Advantageously, the sum x + y + z has a value from 1 to 10.

When R₂₃ is an R₂₇ hydrocarbon group, it can be long and have from 12 to 22 carbon atoms or be short and have from 1 to 3 carbon atoms.

When R₂₅ is an R₂₉ hydrocarbon group, it preferably has from 1 to 3 carbon atoms.

Advantageously, R₂₄, R₂₆ and R₂₈, which are identical or different, are chosen from saturated or unsaturated and linear or branched C₁₁-C₂₁ hydrocarbon groups and more particularly from saturated or unsaturated and linear or branched C₁₁-C₂₁ alkyl and alkenyl groups.

Preferably, x and z, which may be identical or different, are equal to 0 or 1.

Advantageously, y is equal to 1.

Preferably, r, s and t, which may be identical or different, are equal to 2 or 3, and even more particularly are equal to 2.

The anion X⁻ is preferably a halide, preferably chloride, bromide or iodide, a (C₁-C₄)alkyl sulfate or a (C₁-C₄)alkyl- or (C₁-C₄)alkylarylsulfonate. However, it is possible to use methanesulfonate, phosphate, nitrate, tosylate, an anion derived from an organic acid, such as acetate or lactate, or any other anion that is compatible with the ammonium containing an ester function.

The anion X⁻ is more particularly still chloride, methyl sulfate or ethyl sulfate.

Use is made more particularly, in the composition according to the invention, of the ammonium salts of formula (XII) in which:
- R₂₂ denotes a methyl or ethyl group,
- x and y are equal to 1,
- z is equal to 0 or 1,
- r, s and t are equal to 2,
- R₂₃ is chosen from:
   - the group 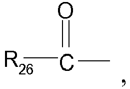
   - methyl, ethyl or C₁₄-C₂₂ hydrocarbon-based groups,
   - a hydrogen atom,
   - R₂₅ is chosen from:
      - the group 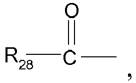
      - a hydrogen atom,
      - R₂₄, R₂₆ and R₂₈, which may be identical or different, are chosen from linear or branched, saturated or unsaturated C₁₃-C₁₇ hydrocarbon-based groups, and preferably from linear or branched, saturated or unsaturated C₁₃-C₁₇ alkyl and alkenyl groups.

Advantageously, the hydrocarbon-based groups are linear.

Among the compounds of formula (XII), examples that may be mentioned include salts, especially the chloride or methyl sulfate, of diacyloxyethyldimethylammonium, diacyloxyethylhydroxyethylmethylammonium, monoacyloxyethyldihydroxyethylmethylammonium, triacyloxyethylmethylammonium or monoacyloxyethylhydroxyethyldimethylammonium, and mixtures thereof. The acyl groups preferably contain 14 to 18 carbon atoms and are obtained more particularly from a plant oil, such as palm oil or sunflower oil. When the compound comprises several acyl groups, the latter can be identical or different.

These products are obtained, for example, by direct esterification of triethanolamine, triisopropanolamine, an alkyldiethanolamine or an alkyldiisopropanolamine, which are optionally oxyalkylenated, with fatty acids or with fatty acid mixtures of plant or animal origin, or by transesterification of the methyl esters thereof. This esterification is followed by a quaternization by means of an alkylating agent such as an alkyl halide, preferably methyl or ethyl halide, a dialkyl sulfate, preferably dimethyl or diethyl sulfate, methyl methanesulfonate, methyl para-toluenesulfonate, glycol chlorohydrin or glycerol chlorohydrin.

Such compounds are sold, for example, under the names Dehyquart® by the company Henkel, Stepanquat® by the company Stepan, Noxamium® by the company Ceca or Rewoquat® WE 18 by the company Rewo-Witco.

The composition according to the invention may contain, for example, a mixture of quaternary ammonium monoester, diester and triester salts with a weight majority of diester salts.

Use may also be made of the ammonium salts comprising at least one ester function described in patents US-A-4 874 554 and US-A-4 137 180.

Use may also be made of behenoylhydroxypropyltrimethylammonium chloride, for example, sold by the company Kao under the name Quartamin BTC 131.

Preferably, the ammonium salts comprising at least one ester function comprise two ester functions.

Among the cationic surfactants, it is more particularly preferred to choose cetyltrimethylammonium, behenyltrimethylammonium and dipalmitoylethylhydroxyethylmethylammonium salts, and mixtures thereof, and more particularly behenyltrimethylammonium chloride, cetyltrimethylammonium chloride, and dipalmitoylethylhydroxyethylammonium methosulfate, and mixtures thereof.

Preferably, when they are present, the surfactant(s) are chosen from nonionic surfactants and cationic surfactants.

When the composition comprises one or more surfactants, their content may preferably range from 0.05% to 20% by weight, more preferentially from 0.1% to 10% by weight and better still from 0.5% to 5% by weight relative to the total weight of the composition.

The composition according to the invention may advantageously comprise one or more silicones or silicone derivatives, in soluble, dispersed or microdispersed form.

It may thus comprise one or more volatile or non-volatile silicones, which are non-organomodified or organomodified with organic groups especially such as amino groups, quaternized amino groups or thiol groups.

The composition according to the invention may also comprise fatty substances, which may be chosen especially from plant, mineral and synthetic oils, non-oxyalkylenated or non-glycerolated C₁₂-C₃₀ fatty alcohols, waxes and ceramides.

The composition according to the invention may also comprise one or more additives chosen from plasticizers, co-thickeners other than those described previously, sunscreens, antioxidants, acids, bases, fragrances, preserving agents, mineral fillers, nacreous agents, opacifiers, dyes and glitter flakes.

A person skilled in the art will take care to select the optional additives and the amounts thereof such that they do not harm the properties of the compositions of the present invention.

These additives may be present in the composition according to the invention in a content ranging from 0 to 20% by weight, relative to the total weight of the composition.

Particularly preferably, the composition according to the invention also comprises water, which advantageously represents from 40% to 99% by weight, preferably from 50% to 95% by weight and better still from 55% to 80% by weight, relative to the total weight of the composition.

The composition can additionally comprise one or more water-soluble organic solvents (solubility of greater than or equal to 5% by weight in water at 25°C and at atmospheric pressure).

Examples of water-soluble organic solvents that may be mentioned include linear or branched and preferably saturated monoalcohols or diols, comprising 2 to 10 carbon atoms, such as ethyl alcohol, isopropyl alcohol, hexylene glycol (2-methyl-2,4-pentanediol), neopentyl glycol and 3-methyl-1,5-pentanediol, butylene glycol, dipropylene glycol and propylene glycol; aromatic alcohols such as phenylethyl alcohol; polyols containing more than two hydroxyl functions, such as glycerol; polyol ethers, for instance ethylene glycol monomethyl, monoethyl or monobutyl ether, propylene glycol or ethers thereof, for instance propylene glycol monomethyl ether; and also diethylene glycol alkyl ethers, especially C₁ to C₄ alkyl ethers, for instance diethylene glycol monoethyl ether or monobutyl ether, alone or as a mixture.

The water-soluble organic solvents, when they are present, generally represent from 1% to 40% by weight and preferably from 5% to 35% by weight relative to the total weight of the composition.

Preferably, in the composition according to the invention, the pH ranges from 3 to 11 and preferably from 4 to 9.

The composition according to the invention may be in the form of liquids that are more or less thickened, gels, creams, pastes or foams.

Preferably, the composition according to the invention is provided in the gel form.

In a preferred alternative form, the viscosity of the compositions of the invention is greater than or equal to 0.1 Pa.s, better still greater than or equal to 0.2 Pa.s and even better still greater than or equal to 0.5 Pa.s at 25°C and at a shear rate of 1 s⁻¹. This viscosity can be determined with a rheometer having cone-plate geometry.

A subject of the invention is also the use of the composition as defined previously for styling the hair.

Finally, a subject of the invention is a process for treating keratin fibres, preferably the hair, comprising the application of the composition as defined previously to the keratin fibres, with or without final rinsing after an optional leave-on time.

Preferably, the application of the compositions of the invention is a leave-on application.

The composition according to the invention may be applied at room temperature (25°C) or with a supply of heat (from 45 to 230°C).

The examples that follow are given purely as illustrations of the present invention.

### Examples

Compositions A to D below were prepared from the ingredients given in the table below, in grams of starting material (active material in parentheses).

| **Ingredients** | **A** | **B** | **C** | **D** |
|---|---|---|---|---|
| Acrylates/Steareth-20 methacrylate crosslinked copolymer⁽¹⁾ | 8.5 (2.414 AM) | 8.5 (2.414 AM) | 8 (2.272 AM) | 8 (2.272 AM) |
| Acrylates/hydroxyacrylates copolymer⁽²⁾ | 5 (2.362 AM) | 6 (2.835 AM) | 6 (2.835 AM) | 6 (2.835 AM) |
| Vinylpyrrolidone/ dimethylaminoethyl methacrylate copolymer | 12 (2.382 AM) | 15 (2.977 AM) | 12 (2.382 AM) | 12 (2.382 AM) |
| Glycerol | 3 | 3 | 3 | 3 |
| Disodium salt of ethylenediaminetetraacetic acid | 0.1 | 0.1 | 0.1 | 0.1 |
| Sorbitol as an aqueous 70% solution | 2 (1.4 AM) | 2 (1.4 AM) | 2 (1.4 AM) | 2 (1.4 AM) |
| Octane-1,2-diol | 0.5 | 1 | 0.5 | 0.5 |
| Oxyethylenated (40 EO) hydrogenated castor oil | 1 | 1 | 1 | 1 |
| 2-Methyl-4-isothiazolin-3-one at 9.5% in water | 0.1 (0.0095 AM) | - | - | 0.1 (0.0095 AM) |
| Phenoxy-2-ethanol | 0.9 | - | 0.9 | 0.9 |
| Niacinamide (Vitamin B3 or PP) | 0.1 | - | - | - |
| Panthenol (Provitamin B5) | 0.1 | - | - | - |
| Disodium salt of Brilliant Yellow FCF | 0.002 | - | - | - |
| Triethanolamine | 2.1 | 2 | 2 | 2 |
| Ethanol | - | - | 2 | 2 |
| Fragrance | 0.4 | 0.4 | - | 0.3 |
| Deionized water | qs 100 g | qs 100 g | qs 100 g | qs 100 g |

| | | | | |
|---|---|---|---|---|
| (1) Aculyn® 88 sold by the company Röhm & Haas (2) Acudyne® 180 sold by the company Röhm & Haas (3) Copolymer® 845 sold by the company ISP | | | | |

Compositions A to D according to the invention are in the form of a gel.

Compositions A to D according to the invention are used as styling gels at room temperature (25°C) and are applied to clean, dry or manually dried hair, at a rate of 5 g of composition per head.

Compositions A to D according to the invention have improved working qualities, and in particular easier uptake in the hands and better distribution and good spreading of the gel onto the hair.

Furthermore, compositions A to D according to the invention have high styling performance qualities, i.e. good fixing and long-lasting hold of the hairstyle.

Finally, compositions A to D according to the invention leave little or no residue on the surface of the user's hair, scalp and/or clothes, once the compositions have been removed.

## Claims

1. Composition comprising:
i) one or more anionic associative polymers,
ii) one or more anionic fixing polymers,
iii) one or more cationic fixing polymers,
the said anionic associative polymer(s) comprising at least one unit of formula (I) and at least one unit of formula (II) below: 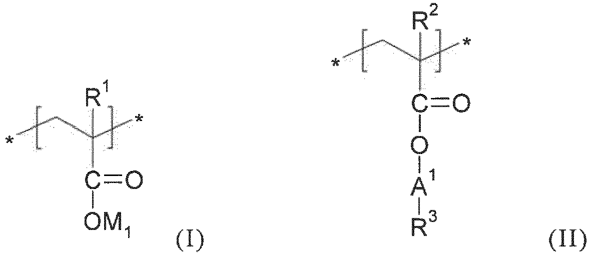
with R¹ and R², independently of each other, representing a hydrogen atom or a methyl group,
R³ representing a C₈-C₃₀ alkyl radical,
M₁ representing a hydrogen atom or a physiologically acceptable cation,
A¹ representing a group -(CH₂CH₂O)ₓ-, x being an integer ranging from 5 to 35, a group -(CH₂CHMeO)_{y}-, y being an integer ranging from 5 to 35, or a group -(CH₂CH₂O)ₓ-(CH₂CHMeO)_{y}-, the sum x + y being an integer ranging from 5 to 35 with x and y being greater than 0,
the said anionic fixing polymer(s) comprising at least one unit of formula (III) and at least one unit of formula (IV) below: 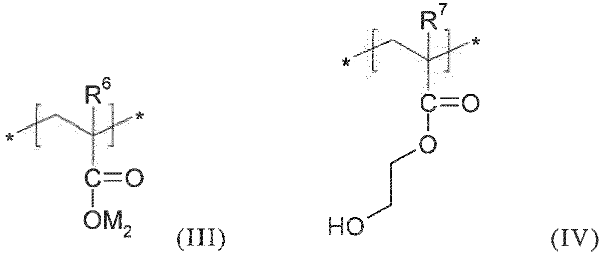
with R⁶ and R⁷, independently of each other, representing a hydrogen atom or a methyl group, and
M₂ representing a hydrogen atom or a physiologically acceptable cation, and
the said cationic fixing polymers are chosen from copolymers derived from acrylic or methacrylic esters comprising:
at least one unit of formulae (VI) and (VII) 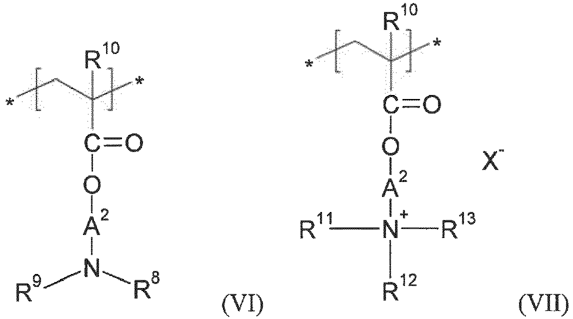 in which:
R⁸ and R⁹, which may be identical or different, represent a hydrogen atom or an alkyl group containing from 1 to 6 carbon atoms;
R¹⁰, which may be identical or different in each formula, denotes a hydrogen atom or a CH₃ radical;
A², which may be identical or different in each formula, denotes a linear or branched alkyl group comprising 1 to 6 carbon atoms or a hydroxyalkyl group comprising 1 to 4 carbon atoms;
R¹¹, R¹² and R¹³, which may be identical or different in each formula, represent an alkyl group containing from 1 to 18 carbon atoms or a benzyl radical;
X⁻ denotes a methosulfate anion or a halide such as chloride or bromide,
- and at least one unit derived from one or more comonomers chosen from vinyllactams.

2. Composition according to Claim 1, **characterized in that** the anionic associative polymer(s) are crosslinked.

3. Composition according to Claim 1 or 2, **characterized in that** the anionic associative polymer(s) also comprise at least one unit of formula (V) below: 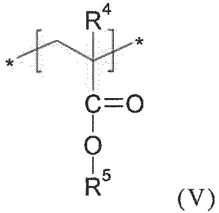
with R⁴ representing a hydrogen atom or a methyl group, and
R⁵ representing a C₁ to C₄ alkyl group, and in particular a methyl or ethyl group.

4. Composition according to any one of the preceding claims, **characterized in that** the anionic associative polymer(s) represent from 0.1% to 20% by weight, preferably from 0.5% to 15% by weight, more preferentially from 1% to 10% by weight and in particular from 1% to 5% by weight, relative to the total weight of the composition.

5. Composition according to any one of the preceding claims, **characterized in that** the anionic fixing polymer(s) also comprise at least one unit of formula (V) below: 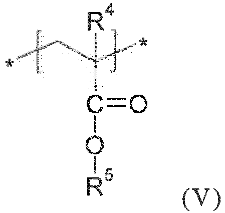
with R⁴ representing a hydrogen atom or a methyl group, and
R⁵ representing a C₁ to C₄ alkyl group, in particular a methyl, ethyl or n-butyl group.

6. Composition according to any one of the preceding claims, **characterized in that** the anionic fixing polymer(s) are non-crosslinked.

7. Composition according to any one of the preceding claims, **characterized in that** the anionic fixing polymer(s) represent from 0.1% to 20% by weight, preferably from 0.2% to 15% by weight, more preferentially from 0.5% to 10% and in particular from 1% to 8% by weight, relative to the total weight of the composition.

8. Composition according to Claim 1, **characterized in that** the cationic fixing polymer(s) are chosen from cationic fixing polymers of family (1), and more particularly from quaternized or non-quaternized, and preferably non-quaternized, vinylpyrrolidone/dialkylaminoalkyl acrylate or methacrylate copolymers.

9. Composition according to Claim 1, **characterized in that** the cationic fixing polymer(s) are chosen from non-quaternized vinylpyrrolidone/dialkylaminoalkyl acrylate or methacrylate copolymers.

10. Composition according to any one of the preceding claims, **characterized in that** the cationic fixing polymer(s) represent from 0.1% to 40% by weight, preferably from 1% to 30% by weight and in particular from 2% to 20% by weight, relative to the total weight of the composition.

11. Use of the composition as defined in any one of the preceding claims, for styling keratin fibres, preferably the hair.

12. Process for treating keratin fibres, preferably the hair, comprising the application of the composition as defined in any one of Claims 1 to 10 to the keratin fibres.

## Patentansprüche

1. Zusammensetzung, umfassend:
i) ein oder mehrere anionische assoziative Polymere,
ii) ein oder mehrere anionische fixierende Polymere,
iii) ein oder mehrere kationische fixierende Polymere,
wobei das anionische assoziative Polymer bzw. die anionischen assoziativen Polymere mindestens eine Einheit der Formel (I) und mindestens eine Einheit der Formel (II) unten umfasst bzw. umfassen: 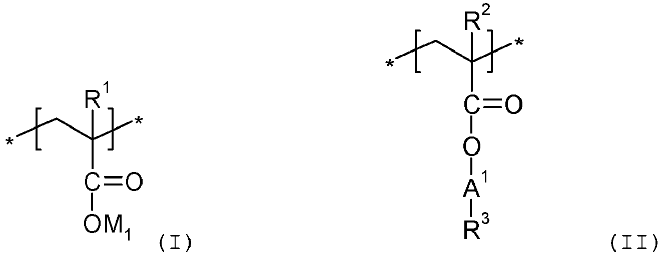
wobei R¹ und R² unabhängig voneinander für ein Wasserstoffatom oder eine Methylgruppe stehen,
R³ für einen C₈-C₃₀-Alkylrest steht,
M₁ für ein Wasserstoffatom oder ein physiologisch unbedenkliches Kation steht,
A¹ für eine Gruppe - (CH₂CH₂O)ₓ-, wobei x für eine ganze Zahl im Bereich von 5 bis 35 steht, eine Gruppe - (CH₂CHMeO) _{y}-, wobei y für eine ganze Zahl im Bereich von 5 bis 35 steht, oder eine Gruppe - (CH₂CH₂O) ₓ-(CH₂CHMeO)_{y}-, wobei die Summe x + y eine ganze Zahl im Bereich von 5 bis 35 ist, wobei x und y größer als 0 sind, steht,
wobei das anionische fixierende Polymer bzw. die anionischen fixierenden Polymere mindestens eine Einheit der Formel (III) und mindestens eine Einheit der Formel (IV) unten umfasst bzw. umfassen: 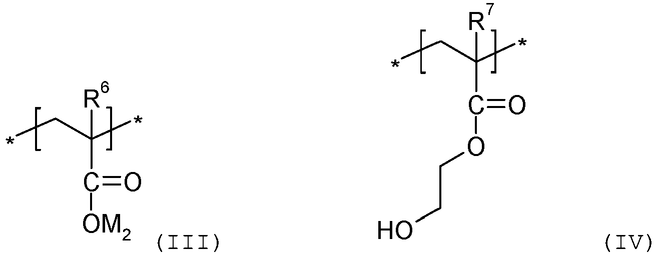
wobei R⁶ und R⁷ unabhängig voneinander für ein Wasserstoffatom oder eine Methylgruppe stehen und
M₂ für ein Wasserstoffatom oder ein physiologisch unbedenkliches Kation steht, und
wobei die kationischen fixierenden Polymere aus Copolymeren ausgewählt sind, die sich von Acrylsäure- oder Methacrylsäureestern ableiten und mindestens eine Einheit der Formeln (VI) und (VII): 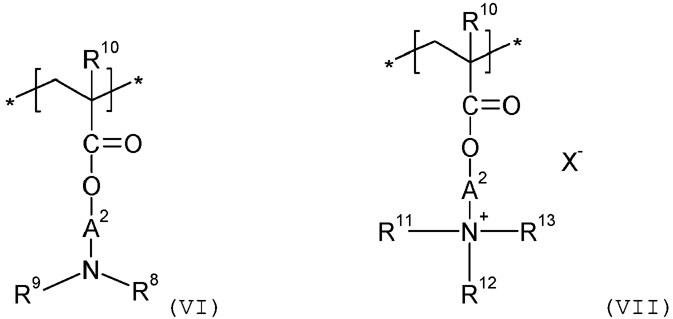 worin:
R⁸ und R⁹, die gleich oder verschieden sein können, für ein Wasserstoffatom oder eine Alkylgruppe mit 1 bis 6 Kohlenstoffatomen stehen;
R¹⁰, das in jeder Formel gleich oder verschieden sein kann, für ein Wasserstoffatom oder einen CH₃-Rest steht;
A², das in jeder Formel gleich oder verschieden sein kann, für eine lineare oder verzweigte Alkylgruppe mit 1 bis 6 Kohlenstoffatomen oder eine Hydroxyalkylgruppe mit 1 bis 4 Kohlenstoffatomen steht;
R¹¹, R¹² und R¹³, die in jeder Formel gleich oder verschieden sein können, für eine Alkylgruppe mit 1 bis 18 Kohlenstoffatomen oder einen Benzylrest stehen;
X⁻ für ein Methosulfat-Anion oder ein Halogenid wie Chlorid oder Bromid steht;
- und mindestens eine Einheit, die sich von einem oder mehreren Comonomeren, die aus Vinyllactamen ausgewählt sind, ableitet,
umfassen.

2. Zusammensetzung nach Anspruch 1, **dadurch gekennzeichnet, dass** das anionische assoziative Polymer bzw. die anionischen assoziativen Polymere vernetzt ist bzw. sind.

3. Zusammensetzung nach Anspruch 1 oder 2, **dadurch gekennzeichnet, dass** das anionische assoziative Polymer bzw. die anionischen assoziativen Polymere außerdem mindestens eine Einheit der Formel (V) unten umfasst bzw. umfassen: 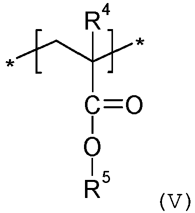
wobei R⁴ für ein Wasserstoffatom oder eine Methylgruppe steht und
R⁵ für eine C₁- bis C₄-Alkylgruppe und insbesondere eine Methyl- oder Ethylgruppe steht.

4. Zusammensetzung nach einem der vorhergehenden Ansprüche, **dadurch gekennzeichnet, dass** das anionische assoziative Polymer bzw. die anionischen assoziativen Polymere 0,1 bis 20 Gew.-%, vorzugsweise 0,5 bis 15 Gew.-%, weiter bevorzugt 1 bis 10 Gew.-% und insbesondere 1 bis 5 Gew.-%, bezogen auf das Gesamtgewicht der Zusammensetzung, ausmacht bzw. ausmachen.

5. Zusammensetzung nach einem der vorhergehenden Ansprüche, **dadurch gekennzeichnet, dass** das anionische fixierende Polymer bzw. die anionischen fixierenden Polymere außerdem mindestens eine Einheit der Formel (V) unten umfasst bzw. umfassen: 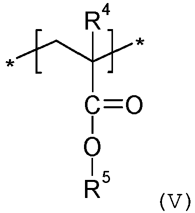
wobei R⁴ für ein Wasserstoffatom oder eine Methylgruppe steht und
R⁵ für eine C₁- bis C₄-Alkylgruppe, insbesondere eine Methyl-, Ethyl- oder n-Butylgruppe steht.

6. Zusammensetzung nach einem der vorhergehenden Ansprüche, **dadurch gekennzeichnet, dass** das anionische fixierende Polymer bzw. die anionischen fixierenden Polymere unvernetzt ist bzw. sind.

7. Zusammensetzung nach einem der vorhergehenden Ansprüche, **dadurch gekennzeichnet, dass** das anionische fixierende Polymer bzw. die anionischen fixierenden Polymere 0,1 bis 20 Gew.-%, vorzugsweise 0,2 bis 15 Gew.-%, weiter bevorzugt 0,5 bis 10 Gew.-% und insbesondere 1 bis 8 Gew.-%, bezogen auf das Gesamtgewicht der Zusammensetzung, ausmacht bzw. ausmachen.

8. Zusammensetzung nach Anspruch 1, **dadurch gekennzeichnet, dass** das kationische fixierende Polymer bzw. die kationischen fixierenden Polymere aus kationischen fixierenden Polymeren der Familie (1) und spezieller aus quaternisierten oder nicht quaternisierten, und nicht quaternisierten, Vinylpyrrolidon/Dialkylaminoalkylacrylat- oder - methacrylat-Copolymeren ausgewählt ist bzw. sind.

9. Zusammensetzung nach Anspruch 1, **dadurch gekennzeichnet, dass** das kationische fixierende Polymer bzw. die kationischen fixierenden Polymere aus nicht quaternisierten Vinylpyrrolidon/Dialkylaminoalkylacrylat- oder - methacrylat-Copolymeren ausgewählt ist bzw. sind.

10. Zusammensetzung nach einem der vorhergehenden Ansprüche, **dadurch gekennzeichnet, dass** das kationische fixierende Polymer bzw. die kationischen fixierenden Polymere 0,1 bis 40 Gew.-%, vorzugsweise 1 bis 30 Gew.-% und insbesondere 2 bis 20 Gew.-%, bezogen auf das Gesamtgewicht der Zusammensetzung, ausmacht bzw. ausmachen.

11. Verwendung der Zusammensetzung gemäß einem der vorhergehenden Ansprüche zum Frisieren von Keratinfasern, vorzugsweise dem Haar.

12. Verfahren zum Behandeln von Keratinfasern, vorzugsweise dem Haar, bei dem man die Zusammensetzung gemäß einem der Ansprüche 1 bis 10 auf die Keratinfasern aufbringt.

## Revendications

1. Composition comprenant :
i) un ou plusieurs polymères associatifs anioniques,
ii) un ou plusieurs polymères fixants anioniques,
iii) un ou plusieurs polymères fixants cationiques,
le ou lesdits polymères associatifs anioniques comprenant au moins un motif de formule (I) et au moins un motif de formule (II) suivantes : 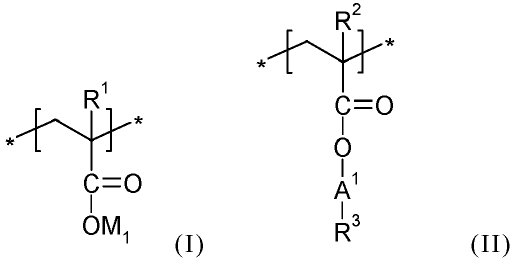
avec R¹ et R² représentant, indépendamment l'un de l'autre un atome d'hydrogène ou un groupe méthyle,
R³ représentant un radical alkyle en C₈ à C₃₀,
M₁ représentant un atome d'hydrogène ou un cation physiologiquement acceptable,
A¹ représentant un groupe -(CH₂CH₂O)ₓ-, x étant un nombre entier variant de 5 à 35, un groupe -(CH₂CHMeO)_{y}-, y étant un nombre entier variant de 5 à 35, ou un groupe -(CH₂CH₂O)ₓ-(CH₂CHMeO)_{y}-, la somme x+y étant un nombre entier variant de 5 à 35 avec x et y supérieurs à 0, et
le ou lesdits polymères fixants anioniques comprenant au moins un motif de formule (III) et au moins un motif de formule (IV) suivantes : 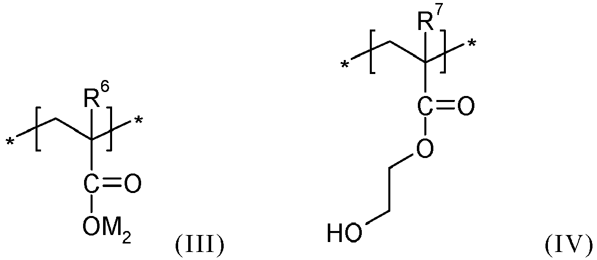
avec R⁶ et R⁷ représentant, indépendamment l'un de l'autre, un atome d'hydrogène ou un groupe méthyle, et
M₂ représentant un atome d'hydrogène ou un cation physiologiquement acceptable, et
le ou lesdits polymères fixants cationiques sont choisis parmi les copolymères parmi les dérivés d'esters acryliques ou méthacryliques et comportant au moins un des motifs de formules (VI) et (VII) suivantes : 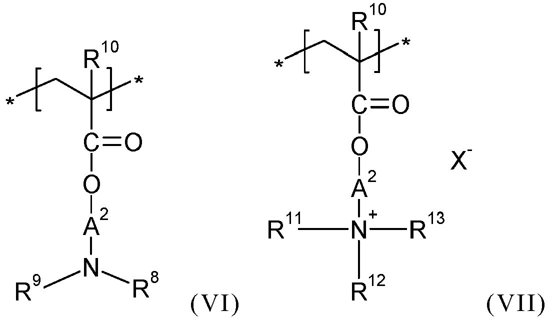 dans lesquelles :
R⁸ et R⁹, identiques ou différents, représentent un atome hydrogène ou un groupe alkyle ayant de 1 à 6 atomes de carbone ;
R¹⁰, identique ou différent dans chaque formule, désigne un atome d'hydrogène ou un radical CH₃ ;
A², identique ou différent dans chaque formule, désigne un groupe alkyle linéaire ou ramifié comportant de 1 à 6 atomes de carbone ou un groupe hydroxyalkyle comportant de 1 à 4 atomes de carbone ;
R¹¹, R¹², R¹³, identiques ou différents dans chaque formule, représentent un groupe alkyle ayant de 1 à 18 atomes de carbone ou un radical benzyle ;
X⁻ désigne un anion méthosulfate ou un halogénure tel que chlorure ou bromure, et
- au moins un motif dérivant d'un ou plusieurs comonomères choisis parmi les vinyllactames

2. Composition selon la revendication 1, **caractérisée en ce que** le ou les polymères associatifs anioniques sont réticulés.

3. Composition selon la revendication 1 ou 2, **caractérisée en ce que** le ou les polymères associatifs anioniques comprennent en outre au moins un motif de formule (V) suivante : 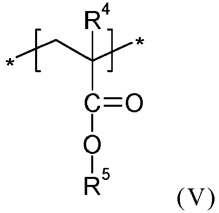
avec R⁴ représentant un atome d'hydrogène ou un groupe méthyle, et
R⁵ représentant un groupe alkyle en C₁ à C₄, et en particulier un groupe méthyle ou éthyle.

4. Composition selon l'une quelconque des revendications précédentes, **caractérisée en ce que** le ou les polymères associatifs anioniques représentent de 0,1 à 20 % en poids, de préférence de 0,5 à 15 % en poids, plus préférentiellement de 1 à 10 % en poids, et en particulier de 1 à 5 % en poids par rapport au poids total de la composition.

5. Composition selon l'une quelconque des revendications précédentes, **caractérisée en ce que** le ou les polymères fixants anioniques comprennent en outre au moins un motif de formule (V) suivante : 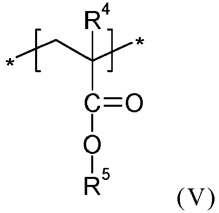
avec R⁴ représentant un atome d'hydrogène ou un groupe méthyle, et
R⁵ représentant un groupe alkyle en C₁ à C₄, en particulier un groupe méthyle, éthyle ou n-butyle.

6. Composition selon l'une quelconque des revendications précédentes, **caractérisée en ce que** le ou les polymères fixants anioniques sont non-réticulés.

7. Composition selon l'une quelconque des revendications précédentes, **caractérisée en ce que** le ou les polymères fixants anioniques représentent de 0,1 à 20 % en poids, de préférence de 0,2 à 15 % en poids, plus préférentiellement de 0,5 à 10 %, et en particulier de 1 à 8 % en poids, par rapport au poids total de la composition.

8. Composition selon l'une quelconque des revendications précédentes, **caractérisée en ce que** le ou les polymères fixants cationiques sont choisis parmi les polymères fixants cationiques de la famille (1), et plus particulièrement parmi les copolymères vinylpyrrolidone/acrylate ou méthacrylate de dialkyl-amino-alkyle quaternisés ou non, et de préférence non quaternisés.

9. Composition selon la revendication 1, **caractérisée en ce que** le ou les polymères fixants cationiques sont choisis parmi les copolymères vinylpyrrolidone/acrylate ou méthacrylate de dialkyl-amino-alkyle non quaternisés.

10. Composition selon l'une quelconque des revendications précédentes, **caractérisée en ce que** le ou les polymères fixants cationiques représentent de 0,1 à 40 % en poids, de préférence de 1 à 30 % en poids, et en particulier de 2 à 20 % en poids, par rapport au poids total de la composition.

11. Utilisation de la composition telle que définie dans l'une quelconque des revendications précédentes pour le coiffage des fibres kératiniques, de préférence des cheveux.

12. Procédé de traitement des fibres kératiniques, de préférence des cheveux, comprenant l'application sur les fibres kératiniques de la composition telle que définie dans l'une quelconque des revendications 1 à 11.
